# EUROPEAN PATENT SPECIFICATION

(11) **EP 2 632 342 B1**
(45) Date of publication and mention of the grant of the patent: **05.10.2016**
(21) Application number: 11782273.4
(22) Date of filing: 19.10.2011
(51) Int. Cl.: A61B 17/00, A61B 17/06, A61B 17/04

(54) **FIBROUS CONTAINMENT FOR HEMOSTASIS PLUG**
FASERIGE VERKAPSELUNG FÜR HÄMOSTASEPFROPF
STRUCTURE D'ENDIGUEMENT FIBREUSE POUR CLOU HÉMOSTATIQUE

(30) Priority: 25.10.2010 US 406426 P
(43) Date of publication of application: 04.09.2013
(73) Proprietor: Boston Scientific Scimed, Inc., Maple Grove, MN 55311 (US)
(72) Inventor: JENSON, Mark L., Greenfield Minnesota 55357 (US); HILL, Jason P., Brooklyn Park Minnesota 55443 (US); FENG, James Q., Maple Grove Minnesota 55369 (US); SOGARD, David J., Edina Minnesota 55424 (US); WARNER, Robert W., Woodbury Minnesota 55125 (US); HAVERKOST, Patrick A., Brooklyn Park Minnesota 55429 (US)
(74) Representative: Vossius & Partner Patentanwälte Rechtsanwälte mbB
(86) International application number: PCT/US2011/056857
(87) International publication number: WO 2012/061017

(56) References cited:
- WO-A1-2010/030933
- US-A- 3 762 418
- US-A- 5 976 162
- US-A1- 2006 229 670

## Description

### Field

The present disclosure relates generally to medical devices and more particularly to methods and devices for closing and/or sealing punctures in tissue.

### Background

Many medical procedures use percutaneous blood vessel access. A typical procedure has the following steps, generally performed in order as listed. A hollow needle is inserted into the artery. An introducer guide wire is inserted through the needle. The needle is pulled out, while the guide wire is held in place so that its tip stays in the artery. A short, follow, tapered dilator and a thin-walled, separate, outer introducer sheath are introduced over the guide wire and into the artery, again while the guide wire is held in place so that its tip stays in the artery. The introducer guide wire and the dilator are pulled out, while the introducer sheath is held so that its tip stays in the artery. A procedural guide wire is inserted through the introducer sheath, while the introducer sheath is held so that its tip stays in the artery. Using the procedural guide wire as a guide, a catheter is inserted into the introducer sheath, while the introducer sheath is held so that its tip stays in the artery.

Once the procedure is completed, the medical devices or other equipment introduced into the vessel can be retracted through the blood vessel, out the opening in the blood vessel wall, and out through the tissue tract to be removed from the body. The physician or other medical technician is presented with the challenge of trying to close the opening in the blood vessel and/or the tissue tract formed in the epidermis and subcutaneous tissue. A number of different device structures, assemblies, and methods are known for closing the opening in the blood vessel and/or tissue tract, each having certain advantages and disadvantages. However, there is an ongoing need to provide new and improved device structures, assemblies, and/or methods for closing and/or sealing the opening in the blood vessel and/or tissue tract.

US 2006/229670 represents the closest prior art and discloses a medical closure system for sealing a puncture through a wall of a blood vessel or wall of a body cavity. US 5 976 162 A discloses a micro-vaso-occlusive device made up of a binder and thrombogenic fibers.

US 3 762 418 A discloses a surgical suture having an eye-less needle swagged to an end of a main suture section. The main suture section ends are fused portions of two separate strands of suture material. The separate strands form an intermediate portion of the main suture section between the fused portions.

### Brief Summary

The invention is defined in the independent claims. Preferred embodiments are defined in the dependent claims.

An embodiment of the present disclosure includes a device for sealing an opening in a vessel wall, including: an anchor configured to be disposed adjacent to an interior surface of the vessel wall adjacent the opening in the vessel wall; a suture having a distal end extending into the anchor and configured to extend proximally through the opening in the vessel wall, the suture including a plurality of filaments that are braided outside the anchor and are unbraided inside the anchor, the filaments extending laterally within the anchor; and a plug disposed over the suture proximal to and adjacent to the anchor, the plug being longitudinally compressible and radially expandable when forced against the anchor.

Another embodiment of the present disclosure includes a device for sealing an opening in a vessel wall, including: an elongate insertion sheath; an anchor disposed within a distal end of the insertion sheath directly adjacent to a distal opening in the insertion sheath; a suture having a distal end within the anchor and extending proximally from the anchor, the suture being braided outside the anchor and unbraided within the anchor; a plug disposed over the suture proximal to the anchor and adjacent to the anchor, the plug being longitudinally compressible and radially expandable when forced against the anchor; a cinch button disposed over the suture proximal the plug and adjacent to the plug, the cinch button frictionally maintaining its longitudinal position along the suture after the plug has been longitudinally compressed against the anchor; a push rod disposed over the suture proximal the cinch button and adjacent to the cinch button, the push rod being configured for delivering a longitudinally compressive force from a proximal end of the suture; and an elongate device sheath disposed over the plug, cinch button and push rod and disposed within the insertion sheath.

An additional embodiment of the present disclosure includes a method for forming a device for sealing an opening in a vessel wall, including: providing a fully braided suture; unbraiding a distal portion of the suture; positioning unbraided filaments of the suture to flare outward from a waist location on the suture; and molding an anchor around the outwardly flared filaments and the waist location.

### Brief Description of the Drawings

Embodiments of the present disclosure may be more completely understood in consideration of the following detailed description of the various embodiments in connection with the accompanying drawings, in which:
FIG. 1 is a side-view cross-sectional schematic drawing of an exemplary device for sealing an opening in a blood vessel;
FIG. 2 is a side-view cross-sectional schematic drawing of an exemplary anchor and suture with filaments that are braided outside the anchor and unbraided inside the anchor;
FIG. 3 is a side-view cross-sectional schematic drawing of an exemplary anchor and suture with filaments that are braided outside the anchor and unbraided inside the anchor, the anchor having a proximally-extending portion that protrudes through the wall of the vessel;
FIG. 4 is a side-view cross-sectional schematic drawing of an exemplary anchor and suture with filaments that are braided outside the anchor and unbraided inside the anchor, with a cinch seal disposed over the suture between the plug and the anchor;
FIG. 5 is a side-view cross-sectional schematic drawing of an exemplary anchor and suture with filaments that are braided outside the anchor and unbraided inside the anchor, where a first subset of the filaments extends around a lateral periphery of the plug and a second subset of the filaments extends generally along a central axis of the plug.
FIG. 6 is a side-view cross-sectional schematic drawing of the sealing device prior to use, as packaged into bypass tube;

While the devices and methods described herein are amenable to various modifications and alternative forms, specifics thereof have been shown by way of example in the drawings and will be described in detail. It should be understood, however, that the intention is not to limit the devices and methods to the particular embodiments described. On the contrary, the intention is to cover all modifications, and alternatives falling within the scope of the claims.

### Detailed Description

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.718, 3, 3.14159265, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The drawings, which are not necessarily to scale, depict illustrative embodiments and are not intended to limit the scope of the invention.

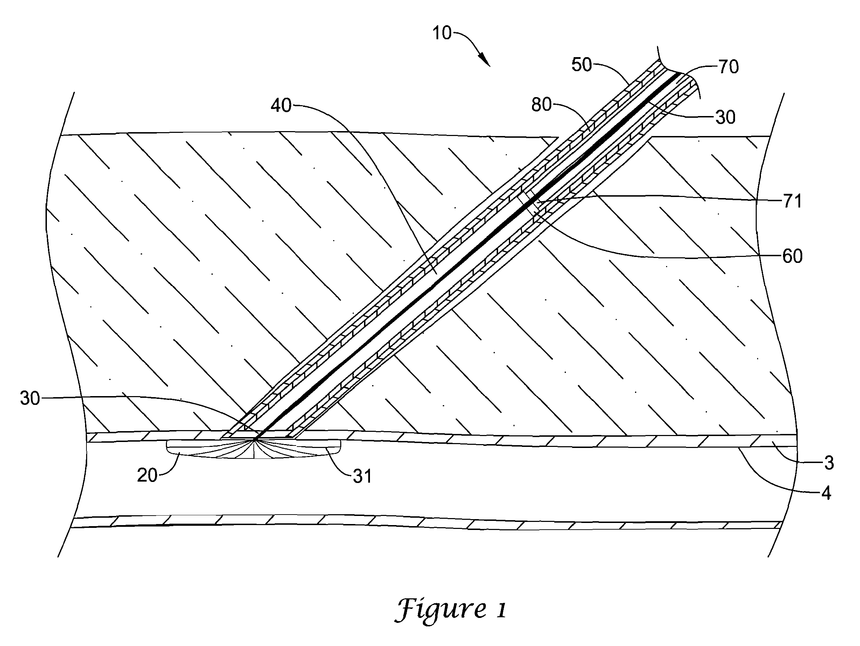

FIG. 1 is a side-view cross-sectional schematic drawing of an exemplary device 10 for closing and/or sealing an opening in a blood vessel and/or adjacent tissue tract that was created to gain access to the vessel to perform a medical procedure.

FIG. 1 shows the various elements at an intermediate stage in the closing/sealing procedure. Initially, once the catheter and other suitable elements from the medical procedure are removed from the vessel through the tract, an anchor 20 is inserted through the tract into the vessel. A suture 30 then pulls the anchor 20 against an inside surface 4 of a wall 3 of the vessel to cover and seal the tract. Once in place, the anchor 20 remains in the vessel against the vessel wall 3, while the suture 30 extends from the anchor 20, through the opening in the vessel wall 3, through the tract, and outside the body of the patient. It is this state that is shown in FIG. 1. In a later stage of the sealing procedure, a plug 40 is longitudinally compressed against the proximal-facing side of the anchor 20, where it expands radially and generally fills the opening in the vessel wall 3. This and other stages are shown in later figures and are described in detail herein.

Many of the other elements shown in FIG. 1 help to deliver and properly engage the anchor 20, suture 30 and plug 40 to the opening in the vessel wall 3. Some of these other elements are typically removed once the anchor 20, suture 30 and plug 40 have been properly placed. The functions of these elements are described briefly here, and are described in greater detail in later figures that show the insertion and positioning process.

An insertion sheath 50 is the first element to be inserted into the opening in the vessel wall 3. Most of the other elements are housed inside the insertion sheath 50 for insertion into the body, and pass through an opening at the distal end of the insertion sheath 50 for deployment. The distal tip of the insertion sheath 50 may be angled away from perpendicular to an axis of the insertion sheath 50, as shown in FIG. 1, so that when engaged, the angled tip and the anchor are both parallel or at least roughly parallel to the wall of the artery. Such an angle for the distal tip may include 0 degrees (making it perpendicular to the axis of the insertion sheath 50), 5 degrees, 10 degrees, 15 degrees, 20 degrees, 25 degrees, 30 degrees, 35 degrees, 40 degrees, 45 degrees, 50 degrees, 55 degrees, 60 degrees, or more than 60 degrees.

A push rod 70 is controllable from the proximal end of the suture 30 or the proximal end of the device 10, and provides a longitudinal force to a cinch button 60 and plug 40 along the suture 30 in the distal direction. A compression bead 71 is made integral with or is attached to the distal end of the push rod 70. A user pushes on the push rod 70, forcing the compression bead 71 against the cinch button 60, forcing the cinch button 60 against the plug 40, and forcing the plug 40 to longitudinally compress against the anchor 20. The push rod 70 may be a coil, a tube, or any other suitable shape that can transmit a force over the suture 30 in the distal direction. In some cases, the compression bead 71 may house a heater coil, which, after the plug 40 is axially compressed and locked in place, can be powered to melt the suture 30 and separate the handle (not shown) from the implanted components.

The cinch button 60 is typically shaped as a disk or washer surrounding the suture 30, which has a significant frictional force with respect to the suture 30. In many cases, the inner diameter of the cinch button 60 is smaller than the nominal diameter of the suture 30. The cinch button 60 may be pushed distally by the compression bead 71 on the distal end of the push rod 70, when the plug 40 is longitudinally compressed. After the plug 40 has been suitably compressed, the push rod 70 and attached compression bead 71 are withdrawn in the proximal direction, while the cinch button 60 remains in place on the suture 30. The elastic spring force of the plug 40 is less than the frictional force between the suture 30 and the cinch button 60, so the cinch button 60 may hold the plug 40 in place in the longitudinally compressed and radially expanded state.

The plug 40 is compressed longitudinally against the anchor 20, which seals the ateriotomy to prevent pressurized blood from entering into the tissue tract. The longitudinal compression also forces the plug material radially outward, which fills the tissue tract and seals the anchor 20 in place. In some cases, the plug 40 may include a material that swells to fill space in the tissue tract and/or opening in the vessel wall 3, such as by elastic expansion, fluid absorption, chemical reaction, as well as other suitable swelling and/or expansion. The plug 40 can be configured to promote hemostasis and/or clotting adjacent to the vessel. The plug 40 may be formed from materials such as a collagen, collagen foam, gelatin foam, PEG, starch powder, a suitable hemostatic material, a suitable clot-promoting material, as well as other suitable material, as desired. In some cases, other materials can be used to provide control of thrombogenicity or hydration. The plug 40 may include a biodegradable material so that, over time, the plug 40 is degraded, eroded, and/or absorbed in the body. In the example of FIG. 1, the plug 40 is generally cylindrical in shape with a lumen extending therethrough. As illustrated, the plug 40 is shown prior to axial compression. In some cases, the plug 40 can include one or more voids, notches, slits, or other modifications to provide a desired axial compression of plug 40. In some cases, the illustrative plug 40 can be processed to have desired expansion characteristics. For example, the plug 40 can be tenderized to break down cell walls to increase the rate of expansion of the plug 40 and reduce the forces necessary to deliver the plug 40 to its final configuration.

A device sheath 80 is slidable within the insertion sheath 50, and houses the plug 40, the cinch button 60, the compression bead 71 and the push rod 70. The insertion sheath 50 may be withdrawn proximally prior to the longitudinal compression of the plug 40.

After the anchor 20 has been placed against the inside surface 4 of the vessel wall 3, but prior to the plug 40 being axially compressed, it is common for the plug 40 to be located as close as possible to the anchor 20. In many cases, the plug 40 is in contact with the anchor 20, prior to longitudinal compression of the plug 40. Having a minimal gap or no gap between the plug 40 and the anchor 20 may be beneficial in reducing or minimizing the potential for hematoma formation or the potential for external bleeding complications during or at the end of the procedure. In these cases, the plug 40 and anchor 20 are in contact before, during and after the procedure.

As an alternative, it is possible to initially have a gap between the anchor 20 and the plug 40, when a particular type of device sheath 80 is used. Unlike a typical sheath 80, a so-called "shoehorn" sheath 80 having one or two score lines or perforations running along its length may be left in place until after plug 40 is longitudinally compressed and radially expanded. The compressed plug 40 splits the "shoehorn" sheath 80 during its compression. Once the plug has been suitably compressed, the "shoehorn" sheath 80 is then removed. The "shoehorn" sheath may also have more than two score lines or perforations. In some cases, the score lines or perforations run generally longitudinally from the distal end of the sheath 80, and extend longitudinally at least as long as the plug 40.

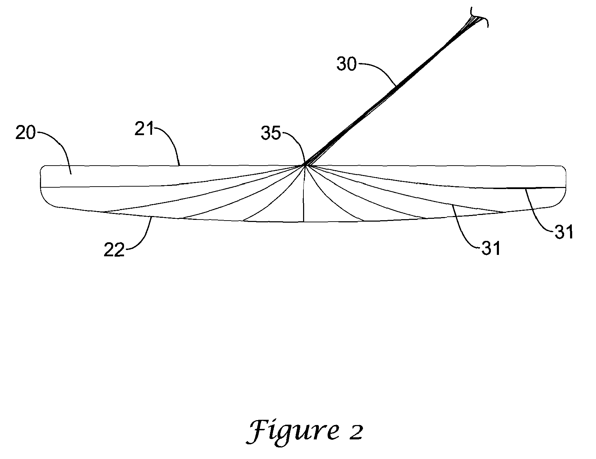

FIG. 2 is a close-up view of the exemplary anchor 20 and suture 30 shown in FIG. 1. The other elements, including the plug 40, are omitted for clarity, although it will be understood that such elements are present in the actual device.

The suture 30 may be considered a multifilar filamentous tension member. The suture 30 may be formed from a spun yarn or other suitable braided or woven material. The suture 30 is braided or woven for much of its length, typically from its proximal end, through the push rod 70, compression bead 71, cinch button 60 and plug 40, to the exterior of the anchor 20. As the suture 30 enters the proximal-facing side 21 of the anchor 20, the suture remains braided. In other words, at the proximal-facing side 21 of the anchor 20, the filaments that make up the suture 30 are radially compressed.

Inside the anchor, 20, the suture 30 may become unwrapped or unbraided at its distal end, so that individual filaments 31 or groups of filaments extend laterally outward toward the distal-facing side of the anchor 20. The filaments 31 flare outward from a waist location 35 along the suture 30 at which the filaments are radially compressed. Note that inside the anchor 20 the filaments 31 do not extend strictly laterally or strictly radially away from the waist location 35, but include a longitudinal component as well, so that they reach the distal-facing side 22 of the anchor 20 in a laterally separated state.

In some cases, the lateral extension of the filaments 31 need not be purely radial; it may include curvature toward or away from a particular axis, an overall spiral (e.g., a pinwheel), or essentially random patterns that progress generally away from the central axis of the suture 30. In these cases, the lateral extent of the filaments 31 inside the anchor may be extended beyond the relatively small lateral extent of the suture 30 itself, thereby increasing the surface area on the anchor 20 on which the attachment force is applied, and beneficially distributing the force over the larger area.

Note that for this document, the term "filament" is used to denote either a single filament or a group of filaments. As such, each filament, as denoted by element number 31 in the figures, may include more than one filament. In general, inside the anchor 20, the suture 30 is unraveled or untangled into two or more filaments 31 that become laterally separated inside the anchor 20. The number of filaments 31 in the suture 30 may be two, three, four, five, six, seven, eight, nine, ten, or more than ten.

The term "braided" is used herein to denote that the individual filaments 31 are interwoven, are knotted, are braided, or are commingled in a manner so that they form a suture 30, a thread, a string, a cable, or other line-like structure that may be stored and handled routinely without unraveling. A typical suture 30 may be considered "braided", as is floss, string, rope, cables, and the like. Likewise, "unbraided" is used to denote that filaments 31 at the distal end of the suture 30 have been unraveled, untangled, or laterally separated in such a manner to individually extend away from each other and/or from a central axis of the suture 30. The filaments 31 may extend laterally individually, or may extend laterally in pairs or groups in the unbraided portion.

There may be advantages to having the filaments 31 laterally separated inside the anchor 20, when compared with attaching a braided suture 30 directly to the anchor 20 without any unbraiding. For instance, because the filaments 31 subtend a larger footprint or area on the anchor 20 than a comparable braided suture, the attachment forces are distributed over a larger area and are therefore more robust.

Note that the suture 30 may be formed by explicitly unraveling a portion of a pre-braided, tangled, or pre-woven element, such as commercially available suture, thread, cable or string. As an alternative, the suture 30 may be formed by winding, weaving, tangling and/or braiding discrete filaments 31 together. The braiding or unbraiding may occur in one or more discrete longitudinal sections along the suture 30. In some cases, the "unbraided" portion of the suture 30 may include filaments 31 that are braided or woven, but are radially expanded away from a central axis of the suture 30, much like the structure of a teepee. These cases may be referred to as "unbraided", even though the filaments 31 remain braided as they flare outward.

In some cases, the proximal face 21 of the anchor 20 is configured to sealingly contact the interior face 4 of the vessel wall 3. In some cases, the anchor 20 is generally oblong-shaped, or shaped as a rounded strip. In some cases, the proximal face 21 of the anchor 20 has a generally rectangular footprint with rounded corners, although other shapes are possible. In some cases, the proximal face 21 of the anchor 20 is elongated along a vessel flow direction. In some cases, the suture 30 enters the anchor 20 at or near the center of the proximal face 21. In some cases, a distal face of the anchor 20 extends from a perimeter of the proximal face 21 and has rounded edges throughout. In some cases, a thickness of the anchor 20 between the proximal and distal faces is greatest along an axis parallel to the vessel flow direction and tapers to both lateral sides of said axis. In some cases, the normal incidence condition may be satisfied for the angular orientation when the suture 30 does not bend upon passing from outside the anchor 20 to inside the anchor 20. In some cases, the proximal face 21 of the anchor 20 includes a bump, and the suture 30 enters the bump toward a heel end of the bump.

The attachment between the anchor 20 and the filaments 31 of the suture 30 can be formed in a number of ways. For example, all or a portion of the anchor 20 may be cast or molded to incorporate the laterally spaced-apart filaments 31. As another example, the anchor 20 may have a shaped cavity, with individual filaments 31 or groups of filaments 31 introduced into portions of the cavity, followed by thermal, chemical or pressure forming to close the cavity and capture the filaments 31. As still another example, the anchor 20 may include multiple components that are assembled with the individual filaments 31 placed between them, followed by post-processing by a combination of thermal, chemical, pressure, solvent, adhesive or other bonding agents to fasten the components together. The filaments 31 may also be attached to the anchor 20 by linearly diverging filaments 31 or braided sub-strands through the thickness of the anchor 20 and injection molding them in place.

Typically, elements such as the intravascular anchor 20, the suture 30 and its filaments 31 are formed from biodegradable materials so that, over time, the elements degrade, erode, and/or are absorbed in the body. Suitable materials for the elements include combinations of PLGA, PLLA, PGA, collagens, glycols, sugars, starches, polysaccharides, polyanhydrides, polycaprolactone, as well as other proteins, analogs, and protein-derived materials. In some cases, the elements may include a combination of the previously mentioned materials to impart a predetermined strength and/or degradation time profile. It is worthwhile to discuss the material construction of the anchor 20 itself.

In general, an intravascular element, the anchor 20, is used to ensure that an extravascular element, the plug 40, is held in place reliably to create hemostasis. A known anchor is made from PLGA (50:50). The known anchor is biodegradable, but commonly takes four weeks to lose 50 percent of its mass, and eight weeks to lose 90 percent of its mass. This decomposition or degradation time is rather long, and in some cases may raise concerns about possible vascular complications that can arise if the known anchor falls into the blood stream during the decomposition or degradation process.

To reduce the probability of complications of this nature, it is desirable that the anchor 20 be able to create hemostasis but not cause ischemia if embolized. In other words, if the anchor 20 should break loose in the blood stream, it should not restrict local blood flow.

One solution is to make the anchor 20 from material(s) that dissolve, decompose, and/or degrade quickly. Fast-degrading materials can include low-molecular-weight saccharides or other suitable material. However, such materials can lack the mechanical strength required for the anchor 20.

A better solution is to blend fibers or nanofibers of materials having adequate mechanical strength into materials that dissolve quickly. An anchor 20 formed from such a blend of materials may achieve the simultaneous goals of a high dissolution speed and a high mechanical strength within a specified time period. The fiber materials themselves may not have a particularly high degradation rate, but the large surface area-to-volume ratio of their fibrous shape and the small geometric size of the fibers may accelerate the material degradation and may reduce or eliminate embolization effects.

The fibers themselves may be electrospun fibers, typically with diameters less than one micron (0.001 mm). In comparison, red blood cells typically have diameters of about five microns (0.005 mm). A specific example includes nano-fibers of PLGA, blended into a quick-dissolving anchor material, such as low-molecular-weight saccharides. The resulting fiber-enforced composite material may be then be loaded into an appropriately shaped mold to produce an anchor 20 with desirable mechanical strength and having quick dissolution characteristics. One possible way for reducing or eliminating embolization effects includes chopping up the fibers into very short lengths, on the order of a few microns long, and distributing them uniformly throughout the rapidly dissolving material.

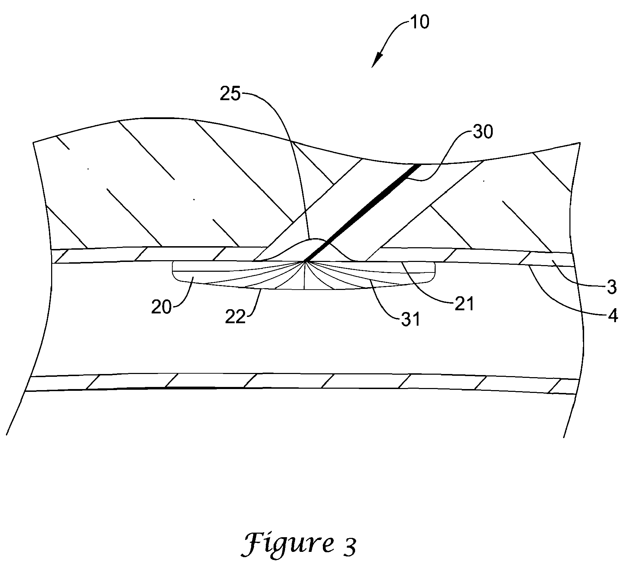

The anchor 20 may have a shape that varies from that of a rounded strip. For instance, FIG. 3 shows an anchor 20 having a proximally-extending portion 25 that protrudes through the wall 3 of the vessel. The proximally extending portion 25 may be referred to as a proximal protrusion, a proximal extension, or a longitudinal protrusion through the opening in the vessel wall 3. In some cases, such a protrusion 25 may help simplify the positioning of the anchor 20 over the hole, due to the self-centering geometry of such a protrusion. In some cases, the protrusion 25 may help increase the strength of the bond between the anchor 20 and the suture 30. Finally, in some cases, the vessel wall 3 may flare outward around the opening, and the proximal protrusion 25 may improve the seal between the anchor 20 and the vessel wall 3 by more closely matching the shape of the vessel wall 3 proximate the opening.

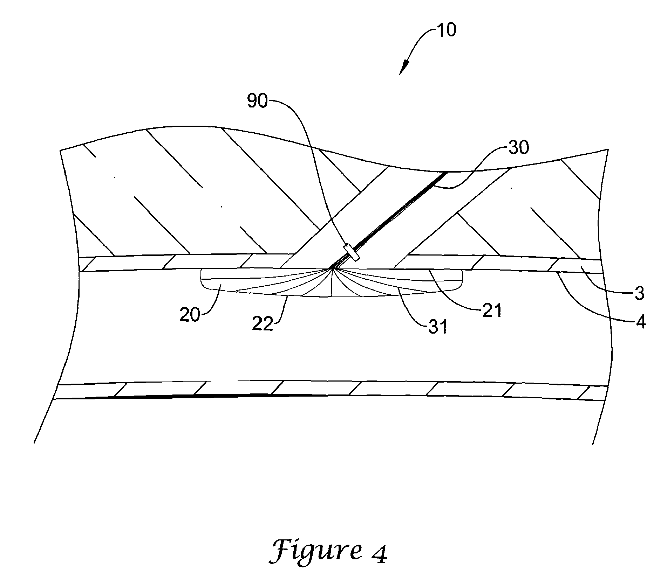

Although the plug 40 is typically directly adjacent to the anchor 20, with no other elements between them and little or no gap between them along the suture 30, there may be instances when another element is disposed on the suture 30 between the plug 40 and the anchor 20. For instance, FIG. 4 shows a so-called "cinch seal" 90 disposed over the suture 30 between the plug (not shown in FIG. 4) and the anchor 20. Such a cinch seal 90 may be shaped like a washer, and may help radially constrain the suture 30 proximal to the anchor 20. In some cases, the cinch seal 90 has the frictional positioning characteristics described above for the cinch button 60. In some cases, the cinch seal 90 is shaped like a dome for a flat-top anchor and may help seal the arteriotomy.

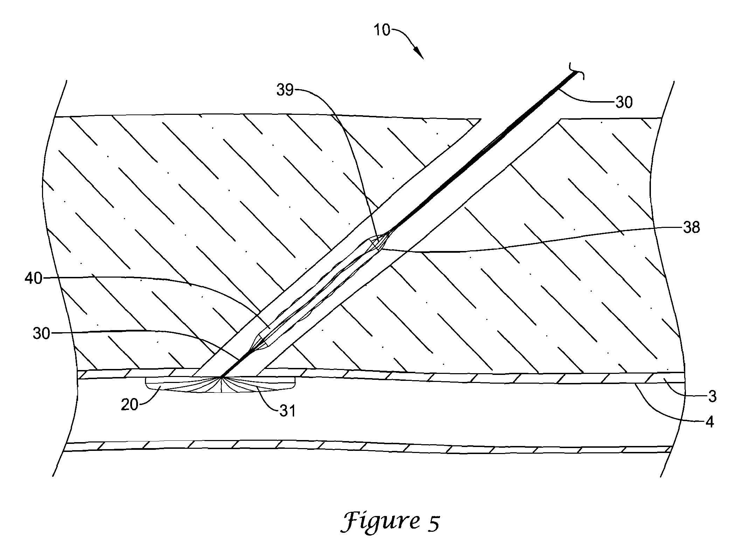

There may be potential advantages to controlling, containing and/or strengthening the plug 40 with the suture 30. For instance, typical hemostasis plugs 40 used in anchor/plug/cinch style arteriotomy closure devices may be stiff or slow to degrade. One can use softer, more absorbent materials that can fill the space more quickly, but materials that are too soft or that absorb water and soften too quickly may not have the strength or stability to stay in the desired shape and position. It is generally impractical to introduce new materials or new elements, since these increase cost and complexity, and may raise different biodegradation or safety issues. Using the suture 30 itself to control, contain and/or strengthen the plug 40 may be advantageous in these cases. An example of such a suture 30 is shown in FIG. 5.

As an alternative to the suture 30 passing through the central axis of the plug 40, as shown in FIG. 1, the suture 30 may have its filaments 31 divided into two subsets, where one subset 38 extends around a lateral periphery of the plug 40 and a second subset 39 extends generally along a central axis of the plug 40.

The center subset 39 of filaments or braids may be relatively strong and may help to keep the plug 40 centrally aligned within the outer subset 38 of filaments during axial collapse of the plug 40. In addition, the outer subset 38 of filaments may allow the use of weaker plug materials, such as gelatin foam, especially when hydrated. In some cases, gelatin foam may be preferable to collagen foam because it has a more rapid bio-absorption time.

Note that the outer subset 38 of filaments can move relative to the center subset 39 of filaments, so that the plug 40 can be compressed without damage.

Note also that for the configuration of FIG. 5, it is desirable to keep the distal end of the plug 40 as close as possible to the anchor 20, in order to avoid forming a gap that, in some cases, may lead to bleeding complications or hematoma formation.

Although the outer subset 38 of the suture 30 is shown in FIG. 5 as being relatively loosely woven, it is typical to use a more densely woven subset of fibers. In general, the outer subset 38 of the suture 30 acts as a fibrous cage that keeps the plug 40 together as a coherent mass. Such a cage typically has fibers that are closely spaced to prevent the plug 40 from escaping laterally as it is longitudinally compressed. Such a fibrous cage may also be in a braided state, although more loosely braided than the center subset 39 of filaments or the remainder of the suture 30. In some cases, the cross-over points in the braid may be fused together, thereby enhancing its ability to function like a cage.

In some cases, to avoid possible jamming at the proximal end of the plug 40 as a cinch button is deployed, the outer subset 38 of the suture may extend proximally around the perimeter of the cinch button. Such a geometry is analogous to the collapse of a Chinese lantern. Alternatively, another cinch button may be included directly proximal to the plug 40 but distal to the point where the outer subset 38 flares outward from the center subset 39. In some cases, to maintain the cage-like function of the outer subset 38, the length of the outer subset may be matched to the changing outer perimeter length of the plug 40.

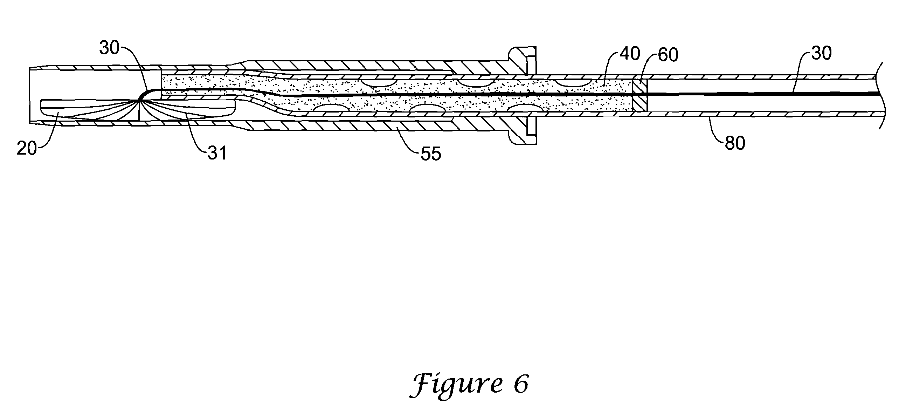

FIG. 6 is a side-view cross-sectional schematic drawing of the sealing device prior to use, as packaged into bypass tube 55.

At the distal end of the bypass tube 55, proximate the distal opening in the bypass tube 55, is the anchor 20. Here, the anchor has been turned about 90 degrees away from its deployed orientation, and fits sideways into the bypass tube 55. The suture 30 exits the anchor 20 in a generally lateral direction, with respect to the bypass tube 55, then turns about 90 degrees to point in a generally longitudinal direction. The distal end of the plug 40 is pushed to one side of the bypass tube 55 to accommodate the anchor 20. In the design of FIG. 6, the plug 40 is shown as having numerous notches or cut-out portions along its exterior; in general, any suitable plug 40 may be used. The distal end of the device sheath 80 is also pushed to one side of the bypass tube to accommodate the anchor. The cinch button 60 is also shown in FIG. 6. As with the design of FIG. 1, the plug 40 and cinch button 60 are pushed distally out of the device sheath 80 (and/or, equivalently, the device sheath 80 is withdrawn proximally over the plug 40 and cinch button 60) prior to or after longitudinal compression of the plug 40.

It should be understood that this disclosure is, in many respects, only illustrative. Changes may be made in details, particularly in matters of shape, size, and arrangement of steps without exceeding the scope of the invention. The invention's scope is, of course, defined by the appended claims.

## Claims

1. A device (10) for sealing an opening in a vessel wall (3), comprising:
an anchor (20) configured to be disposed adjacent to an interior surface (4) of the vessel wall (3) adjacent the opening in the vessel wall (3) and configured to remain in the vessel once in place, the anchor (20) being biodegradable;
a suture (30) having a distal end extending into the anchor (20) and configured to extend proximally through the opening in the vessel wall (3), the suture (30) including a plurality of filaments (31) that are braided outside the anchor (20) and are unbraided inside the anchor (20), the filaments (31) extending outward from a central axis of the suture (30) within the anchor (20); and
a generally cylindrical plug (40, 70) disposed over the suture (30) proximal to and adjacent to the anchor (20), the plug (40, 70) being compressible in its axial direction and expandable in its radial direction when forced against the anchor (20).

2. The device (10) of claim 1, wherein the filaments (31) of the suture (30) extend from a waist location (35) along the suture (30) at which the filaments (31) are compressed towards a central axis of the suture (30).

3. The device of claim 2, wherein the waist location (35) is at a proximal surface of the anchor (20).

4. The device (10) of claim 2, wherein the waist location (35) is within the anchor (20).

5. The device (10) of claim 2, wherein the filaments (31) of the suture (30) are compressed towards the central axis of the suture (30) along essentially the entire suture (30) proximal to the waist location (35).

6. The device (10) of claim 1, wherein proximate the plug (40, 70), all the filaments (31) in the suture (30) extend generally along a central axis of the plug (40, 70).

7. The device (10) of claim 1, wherein proximate the plug (40, 70), a first subset (38) of the filaments (31) extends around a lateral periphery of the plug (40, 70) and a second subset (39) of the filaments (31) extends generally along a central axis of the plug (40, 70).

8. The device (10) of claim 1, wherein the anchor (20) includes a proximally-extending portion (25) configured to protrude through the wall (3) of the vessel.

9. The device (10) of claim 1, wherein the anchor (20) is shaped as a rounded strip, a proximal face (21) of the anchor being configured to sealingly contact the vessel wall (3), the proximal face (21) having a generally rectangular footprint with rounded corners and being elongated along a vessel flow direction, a distal face (22) of the anchor (20) extending from a perimeter of the proximal face (21) and having rounded edges throughout, a thickness of the anchor (20) between the proximal and distal faces being greatest along an axis parallel to the vessel flow direction and tapering to both lateral sides of said axis.

10. The device (10) of claim 1, wherein the anchor (20) is formed from fibers of a mechanically strong material blended into a quick-dissolving material.

11. A method for forming a device (10) for sealing an opening in a vessel wall (3), comprising:
providing a fully braided suture (30);
unbraiding a distal portion of the suture (30);
positioning unbraided filaments (31) of the suture (30) to flare outward from a waist location (35) on the suture (30);
molding an anchor (20) around the outwardly flared filaments (31) and the waist location (35);
disposing a generally cylindrical plug (40, 70) over the suture (30) proximal to and adjacent to the anchor (20), the plug being compressible in its axial direction and expandable in its radial direction when forced against the anchor (20).

12. The method of claim 11, wherein the anchor (20) is formed from fibers of a mechanically strong material blended into a quick-dissolving material.

13. The method of claim 11, wherein the anchor (20) is oblong-shaped, or wherein the anchor (20) is shaped as a rounded strip, a proximal face (21) of the anchor (20) being configured to sealingly contact the vessel wall (3), the proximal face (21) having a generally rectangular footprint with rounded corners and being elongated along a vessel flow direction, a distal face (22) of the anchor (20) extending from a perimeter of the proximal face (21) and having rounded edges throughout, a thickness of the anchor between the proximal and distal faces being greatest along an axis parallel to the vessel flow direction and tapering to both lateral sides of said axis.

14. The method of claim 11, wherein the step of unbraiding a distal portion of the suture (30) comprises:
severing at least one filament (31) of the suture; and
unraveling the suture proximate the at least one severed filament.

## Patentansprüche

1. Vorrichtung (10) zum Verschließen einer Öffnung in einer Gefäßwand (3), die aufweist:
einen Anker (20), der konfiguriert ist, benachbart zu einer Innenfläche (4) der Gefäßwand (3) benachbart der Öffnung in der Gefäßwand (3) angeordnet zu werden und im Gefäß zu bleiben, sobald er sich an Ort und Stelle befindet, wobei der Anker (20) biologisch abbaubar ist;
eine Naht (30), die ein distales Ende aufweist, das sich in den Anker (20) erstreckt und konfiguriert ist, sich proximal durch die Öffnung in der Gefäßwand (3) zu erstrecken, wobei die Naht (30) mehrere Filamente (31) umfasst, die außerhalb des Ankers (20) geflochten sind und innerhalb des Ankers (20) nicht geflochten sind, wobei sich die Filamente (31) von einer Mittelachse der Naht (30) innerhalb des Ankers (20) nach außen erstrecken; und
einen im Allgemeinen zylindrischen Pfropf (40, 70), der über der Naht (30) proximal und benachbart zum Anker (20) angeordnet ist, wobei der Pfropf (40, 70) in seine Achsenrichtung zusammendrückbar und in seine Radialrichtung ausdehnbar ist, wenn er gegen den Anker (20) gedrückt wird.

2. Vorrichtung (10) nach Anspruch 1, wobei sich die Filamente (31) der Naht (30) von einer Einschnürungsstelle (35), an der die Filamente (31) zu einer Mittelachse des Nahtmaterials (30) zusammengedrückt sind, längs der Naht (30) erstrecken.

3. Vorrichtung nach Anspruch 2, wobei sich die Einschnürungsstelle (35) an einer proximalen Oberfläche des Ankers (20) befindet.

4. Vorrichtung (10) nach Anspruch 2, wobei sich die Einschnürungsstelle (35) innerhalb des Ankers (20) befindet.

5. Vorrichtung (10) nach Anspruch 2, wobei die Filamente (31) der Naht (30) im Wesentlichen längs der gesamten Naht (30) proximal zur Einschnürungsstelle (35) zur Mittelachse der Naht (30) zusammengedrückt sind.

6. Vorrichtung (10) nach Anspruch 1, wobei sich in der Nähe des Pfropfs (40, 70) alle Filamente (31) im Nahtmaterial (30) im Allgemeinen längs einer Mittelachse des Pfropfs (40, 70) erstrecken.

7. Vorrichtung (10) nach Anspruch 1, wobei sich in der Nähe des Pfropfs (40, 70) eine erste Teilmenge (38) der Filamente (31) um einen lateralen Umfang des Pfropfs (40, 70) erstreckt und sich eine zweite Teilmenge (39) der Filamente (31) im Allgemeinen längs einer Mittelachse des Pfropfs (40, 70) erstreckt.

8. Vorrichtung (10) nach Anspruch 1, wobei der Anker (20) einen sich proximal erstreckenden Abschnitt (25) aufweist, der konfiguriert ist, durch die Wand (3) des Gefäßes vorzustehen.

9. Vorrichtung (10) nach Anspruch 1, wobei der Anker (20) als ein abgerundeter Streifen geformt ist, wobei eine proximale Fläche (21) des Ankers konfiguriert ist, abdichtend die Gefäßwand (3) zu berühren, wobei die proximale Fläche (21) eine im Allgemeinen rechteckige Grundfläche mit abgerundeten Ecken aufweist und längs einer Gefäßflussrichtung länglich ist, wobei sich eine distale Fläche (22) des Ankers (20) von einem Umfang der proximalen Fläche (21) erstreckt und überall abgerundete Kanten aufweist, wobei eine Dicke des Ankers (20) zwischen der proximalen und distalen Fläche längs einer zur Gefäßflussrichtung parallelen Achse am größten ist und sich zu beiden lateralen Seiten der Achse verjüngt.

10. Vorrichtung (10) nach Anspruch 1, wobei der Anker (20) aus Fasern eines mechanisch starken Materials ausgebildet ist, die in ein schnelllösliches Material gemischt sind.

11. Verfahren zum Bilden einer Vorrichtung (10) zum Verschließen einer Öffnung in einer Gefäßwand (3), das aufweist:
Bereitstellen einer vollständig geflochtenen Naht (30);
Entflechten eines distalen Abschnitts der Naht (30);
Anordnen von entflochtenen Filamenten (31) der Naht (30), so dass sie sich von einer Einschnürungsstelle (35) an der Naht (30) nach außen ausweiten;
Formen eines Ankers (20) um die nach außen ausgeweiteten Filamente (31) und die Einschnürungsstelle (35);
Anordnen eines im Allgemeinen zylindrischen Pfropfs (40, 70) über der Naht (30) proximal und benachbart zum Anker (20), wobei der Pfropf in seine Achsenrichtung zusammendrückbar und in seine Radialrichtung ausdehnbar ist, wenn er gegen den Anker (20) gedrückt wird.

12. Verfahren nach Anspruch 11, wobei der Anker (20) aus Fasern eines mechanisch starken Materials gebildet wird, die in ein schnelllösliches Material gemischt sind.

13. Verfahren nach Anspruch 11, wobei der Anker (20) rechteckig geformt ist oder wobei der Anker (20) als ein abgerundeter Streifen geformt ist, wobei eine proximale Fläche (21) des Ankers (20) konfiguriert ist, abdichtend die Gefäßwand (3) zu berühren, wobei die proximale Fläche (21) eine im Allgemeinen rechteckige Grundfläche mit abgerundeten Ecken aufweist und längs einer Gefäßflussrichtung länglich ist, wobei sich eine distale Fläche (22) des Ankers (20) von einem Umfang der proximalen Fläche (21) erstreckt und überall abgerundete Kanten aufweist, wobei eine Dicke des Ankers zwischen der proximalen und distalen Fläche längs einer zur Gefäßflussrichtung parallelen Achse am größten ist und sich zu beiden lateralen Seiten der Achse verjüngt.

14. Verfahren nach Anspruch 11, wobei der Schritt des Entflechtens eines distalen Abschnitts der Naht (30) aufweist:
Abtrennen mindestens eines Filaments (31) der Naht; und
Zerfasern der Naht in der Nähe des mindestens einen abgetrennten Filaments.

## Revendications

1. Dispositif (10) pour l'obturation d'une ouverture dans une paroi de vaisseau (3), comprenant :
un ancrage (20) prévu pour être mis en place de manière adjacente à une surface intérieure (4) de la paroi de vaisseau (3) adjacente à l'ouverture dans la paroi de vaisseau (3), et prévu pour rester dans le vaisseau une fois mis en place, ledit ancrage (20) étant biodégradable ;
un fil de suture (30) ayant une extrémité distale s'étendant dans l'ancrage (20) et prévu pour s'étendre proximalement dans l'ouverture de la paroi de vaisseau (3), ledit fil de suture (30) comportant une pluralité de filaments (31) tressés à l'extérieur de l'ancrage (20) et non tressés à l'intérieur de l'ancrage (20), lesdits filaments (31) s'étendant extérieurement à un axe central du fil de suture (30) à l'intérieur de l'ancrage (20) ; et
un bouchon (40, 70) sensiblement cylindrique disposé sur le fil de suture (30) de manière proximale et adjacente à l'ancrage (20), ledit bouchon (40, 70) étant compressible dans sa direction axiale et expansible dans sa direction radiale quand il est contraint contre l'ancrage (20).

2. Dispositif (10) selon la revendication 1, où les filaments (31) du fil de suture (30) s'étendent d'un point de serrage (35) le long du fil de suture (30), où les filaments (31) sont comprimés, vers un axe central du fil de suture (30).

3. Dispositif selon la revendication 2, où le point de serrage (35) est sur une surface proximale de l'ancrage (20).

4. Dispositif (10) selon la revendication 2, où le point de serrage (35) est à l'intérieur de l'ancrage (20).

5. Dispositif (10) selon la revendication 2, où les filaments (31) du fil de suture (30) sont comprimés vers l'axe central du fil de suture (30) sensiblement le long de tout le fil de suture (30), proximalement au point de serrage (35).

6. Dispositif (10) selon la revendication 1, où à proximité du bouchon (40, 70), tous les filaments (31) du fil de suture (30) s'étendent sensiblement le long d'un axe central du bouchon (40, 70).

7. Dispositif (10) selon la revendication 1, où à proximité du bouchon (40, 70), un premier sous-ensemble (38) de filaments (31) s'étend autour d'une périphérie latérale du bouchon (40, 70) et un deuxième sous-ensemble (39) de filaments (31) s'étend sensiblement le long d'un axe central du bouchon (40, 70).

8. Dispositif (10) selon la revendication 1, où l'ancrage (20) comprend une section à extension proximale (25) prévue pour faire saillie au travers de la paroi (3) du vaisseau.

9. Dispositif (10) selon la revendication 1, où l'ancrage (20) est formé comme une bande arrondie, une face proximale (21) de l'ancrage étant prévue pour contacter de manière étanche la paroi de vaisseau (3), la face proximale (21) ayant une forme sensiblement rectangulaire avec des coins arrondis et étant allongée dans une direction de flux du vaisseau, une face distale (22) de l'ancrage (20) s'étendant depuis un périmètre de la face proximale (21) et ayant des bords généralement arrondis, l'ancrage (20) présentant une épaisseur entre les faces proximale et distale maximale le long d'un axe parallèle à la direction de flux du vaisseau et s'amincissant sur les deux côtés latéraux de l'axe.

10. Dispositif (10) selon la revendication 1, où l'ancrage (20) est constitué de fibres, d'un matériau mécaniquement résistant mélangé à un matériau à dissolution rapide.

11. Procédé de formation d'un dispositif (10) pour l'obturation d'une ouverture dans une paroi de vaisseau (3), comprenant :
la préparation d'un fil de suture (30) entièrement tressé ;
le détressage d'une partie distale du fil de suture (30) ;
la disposition de filaments (31) non tressés du fil de suture (30) de manière à s'évaser vers l'extérieur depuis un point de serrage (35) sur le fil de suture (30) ;
le moulage d'un ancrage (20) autour des filaments (31) évasés vers l'extérieur et du point de serrage (35) ;
la disposition d'un bouchon (40, 70) sensiblement cylindrique sur le fil de suture (30) de manière proximale et adjacente à l'ancrage (20), ledit bouchon étant compressible dans sa direction axiale et expansible dans sa direction radiale quand il est contraint contre l'ancrage (20).

12. Procédé selon la revendication 11, où l'ancrage (20) est constitué de fibres, d'un matériau mécaniquement résistant mélangé à un matériau à dissolution rapide.

13. Procédé selon la revendication 11, où l'ancrage (20) est de forme oblongue, ou bien où l'ancrage (20) est formé comme une bande arrondie, une face proximale (21) de l'ancrage (20) étant prévue pour contacter de manière étanche la paroi de vaisseau (3), la face proximale (21) ayant une forme sensiblement rectangulaire avec des coins arrondis et étant allongée dans une direction de flux du vaisseau, une face distale (22) de l'ancrage (20) s'étendant depuis un périmètre de la face proximale (21) et ayant des bords généralement arrondis, l'ancrage (20) présentant une épaisseur entre les faces proximale et distale maximale le long d'un axe parallèle à la direction de flux du vaisseau et s'amincissant sur les deux côtés latéraux de l'axe.

14. Procédé selon la revendication 11, où l'étape de détressage d'une partie distale du fil de suture (30) comprend :
le sectionnement d'au moins un filament (31) du fil de suture ; et
le dénouage du fil de suture à proximité dudit au moins un filament sectionné.
